# EUROPEAN PATENT SPECIFICATION

(11) **EP 1 512 377 B1**
(45) Date of publication and mention of the grant of the patent: **14.01.2009**
(21) Application number: 03019627.3
(22) Date of filing: 04.09.2003
(51) Int. Cl.: A61B 17/12

(54) **Apparatus for manipulation of threads such as ligatures**
Einrichtung zur Manipulation von Fäden, wie z.B. Ligaturen
Dispositif de manipulation des fils, par exemple des ligatures

(43) Date of publication of application: 09.03.2005
(73) Proprietor: Friedrich-Alexander-Universität Erlangen-Nürnberg, 91054 Erlangen (DE)
(72) Inventor: Kraus, Hubert, Dr., 92431 Neunburg (DE)
(74) Representative: Vossius & Partner

(56) References cited:
- EP-A- 0 792 621
- DE-A- 19 706 529
- US-A- 3 470 875
- US-A- 4 011 873
- US-A- 4 935 027
- US-A- 5 071 428

## Description

The present invention relates to the field of surgical apparatus, system and method particularly for the placement and controlled manipulation of threads (suture threads) like in ligatures.

For many surgical operations it is necessary to ligate blood or lymph vessels, anatomical channels, hollow organs or soft part tissues by means of a thread. This method, i.e., ligate a thread around a vessel and interlock firmly, should not be underestimated by its importance. It occurs not rarely that a patient must be operated again after a successful operation because of the insufficiency of only one ligature and the bleeding result from it. Thus, an experienced surgeon makes sure that each ligature knot is implemented accurately. Thus, this accurate operation/process is very time-consuming. In many standard interferences, e.g. the thyroid removal, the time for applying ligatures takes a considerable large part of operation time. Taking the time for applying a ligature approximately to be one minute and the number of ligatures to be about 15 to 40 for a thyroid operation, the time just for applying ligatures is in the range of 15 to 40 minutes.

To understand the procedure with the working steps of applying ligatures in a conventional way, this procedure will be explained in more detail in the following. The preparation of the structure where the ligature will take place occurs in standard techniques generally with a preparation forceps or an overholt. Thereby, the connective tissue is pushed or pressed apart by the jaws of the forceps which is arranged perpendicularly to the blood vessel. This process is repeated until the connective tissue is pushed apart parallel to the vessel and the vessel is freely accessible to pass the overholt under said vessel. On both sides of the designated part, where the vessel should be disconnected, the clamp, forceps or hemostat is clamped to arrest the flow of blood in the vessel. The vessels are clamped before they are severed. When a vessel is surgically severed, a ligature of thread, catgut or silk is tied beneath the clamp.

Afterwards, the structure is cut off. While the clamps are kept by the assistant, a thread which is handled by another clamp is looped around each of the two ends of the vessel. The surgeon is knotting the thread next to the clamp, and, during the knot is tightened, the clamp must be opened at the same time. For this process, a good timing between the assistant and the surgeon is very important. After the knot is tightened securely, the clamps can be removed and the ends of the threads can be cut off. The same process is also necessary for the opposite part of the vessel. This standard procedure involves many working steps like passing the appropriate apparatus, passing the thread, applying of the clamps, cutting the vessel and threads, knotting the threads, and so on. In addition to the large number of working steps, typically three persons are involved, namely the surgeon, the assistant, and a surgical nurse.

Apparatuses for facilitating the placement of sutures, ligatures, etc, during surgical operations are known. Such apparatus are often used in order to extend the reach of the surgeon within the body of the patient, without the surgeon having to have her/his hands in actual contact with the suturing needle or the thread.

A surgical instrument for ligatures according to US 4011 873 consisting of a hollow handle in the inside of which thread spools are mounted rotatably, the hollow handle extending into a tube, the lower end of which has a curved portion, a flattened portion provided in the area of curvature, and exit openings for the unwound threads provided on both sides behind the point of the instrument. However, this instrument does not substitute the working steps for pushing or pressing the tissue around the vessel away for a freely accessible vessel.

In WO 97 35523, an apparatus for facilitating the performance of surgical procedures such as the placement of sutures, ligatures and the like, having a cannula potion and a stylet portion is disclosed. The apparatus is configured to hold a suturing needle, having an attached thread. A related device is disclosed in US 5 387 221 with a needle driving device including a pivotally mounted needle holder for a curved suturing needle.

WO 92 11810 discloses a suture applier comprising a shaft having proximal and distal ends and carrying a length of suture along its axis. The suture has a surgical needle at its distal end and a knotted loop formed proximally of the needle. The needle may be used to suture a wound in tissue and may be tied by passing the needle through the knotted loop and closing the loop by pulling on the slidable handle.

DE-U-197 06 529 is considered to represent the closest prior art and discloses a percutaneous vascular closure system with a guide body comprising a through-bore for a wire. The bore through the guide body stretches from the distal to the proximal end and contains a guide wire from a blood vessel. A positioner fixes the position of the closure system in relation to the blood vessel. Two hollow needles at the distal end form, when closed, a tongs-handle. Two needle-thread guides connected to the proximal ends of the hollow needles pull the thread through. An expander mechanism for opening the tongs-handle is connected to the hollow needles. The expander mechanism has two arms. EP 0792621 relates to similar device for assisting a surgical procedure. Specifically, EP 0792621 relates to a type of clamp which allows a surgeon to capture tissue or bone that is difficult to reach and suture that captured material. More particularly, the device allows a surgeon to easily and accurately guide a needle through tissue or bone, that would otherwise be difficult to suture.

It is therefore an object of the present invention to provide an apparatus (also called: ligateur), a method and/or a system which facilitates the placement of ligatures by performing threads already during preparation, resulting in an easier, faster and/or safer ligature preparation. This object of the invention is achieved with the features of the claims.

The invention is based on the idea to use a preparation instrument which guides suture threads, in the following labelled "threads", for the ligature under or around the structure where the ligature should be applied. Therefore, different embodiments of preparation instruments related to forceps, overholt forces or tweezers with different embodiments of thread guiding means are provided.

In principle, the apparatus of the present invention as defined by appended claim 1 comprises at least two shanks wherein each shank has a proximal and a distal end. the at least two shanks which are hinged connected to each other, and at least one ligature guiding means is associated with each shank for guiding at least one thread substantially along said shank to the direction of the distal end. The distal ends are curved generally perpendicular to the plane defined by the proximal portions of the hinged shanks, and each exit is adapted for dispensing the free end of the ligature generally within the plane of the curved distal end of the respective shank. Thus, a free end of the thread is dispensed preferably through exit openings at the vicinity of the distal end portion. The distal end portion of the shanks comprises preferably a spike or jaw for the preparation and guiding the free end of the thread below the structure where the ligature should be applied. The two shanks are preferably movably connected so that a spatial distance between the distal end portions is variable, i.e., they are hinged such that grasping is possible as is with standard forceps, tweezers or overholts. In a preferred embodiment of the present invention, the two shanks are crossbred connected by a pivot axis, and/or comprise grip members, ring handle or finger rings at the proximal end portions providing an apparatus similar to an overholt. Additionally, a ratchet or spring parts could be mounted between the shanks for fixating the position of the shanks to each other or for providing a supportive force either to push the shanks relative to each other apart or together. Inside or at the jaw parts, a guiding means guides the free end of the thread at the end portion or in the vicinity of the end portion away from that jaws and provides the free ends of the thread. Different embodiments of the present invented apparatus can be provided according to the specific needs, depending on the shank connection, the guiding means at the shanks, the forming of the end portions, the fixation of the thread along the guiding means, the providing of the thread and so on.

The shanks, the guiding means and the duct may be fabricated from any suitable material with hardened aluminium, brass or stainless steel being considered as preferred at least for specific parts. However, other suitable metals or plastic materials may optionally be employed. The apparatus may either be without a thread or already comprise at least one thread which may be a usual commercial thread, catgut or silk for sutures. Furthermore, each shank may also comprise more than one guiding means for guiding one or more threads. Also, guiding of more than one thread in one guiding means is possible with a guiding means of an appropriate design. Moreover, in some situations only one thread is needed even if there are more than two guiding means.

Furthermore, the invention also relates to a cartridge, particularly for application in or at the above apparatus and for providing at least one thread in a thread storage means. Moreover, in addition to the provision of a thread, the cartridge may also provide guiding means along the shanks. The cartridge may be either a permanent part of the apparatus or a removable disposable part which could be handled separately and be attached to the apparatus. Furthermore, the invention also relates to a system which comprises at least one of said cartridge and said apparatus.

The present invention will be further described with reference to the accompanying drawings wherein like parts have the same reference signs, wherein:
- Fig. 1a: is a plan view of the apparatus according to a first embodiment of the present invention;
- Fig. 1b: is a side view of the apparatus of the first embodiment;
- Fig. 2a: is a plan view of an apparatus according to a second embodiment of forceps comprising additional removable thread guiding ducts;
- Fig. 2b: is a side view of the second embodiment;
- Fig. 2c: is a plan view of the third embodiment of the present invention comprising guiding means substantially at the distal end portions of the shanks;
- Figs. 3a, 3b: are a side and plan view, respectively, of an apparatus according to the present invention, in a fourth embodiment of a forceps comprising furthermore a thread storage means for providing thread from a thread spool;
- Figs. 4a, 4b: are a plan and side view, respectively, of an apparatus according to a fifth embodiment of the present invention comprising two cartridges of the present invention for providing thread;
- Fig. 5a: is a plan view of two of said cartridges shown in Figs. 4a and 4b;
- Fig. 5b: is a side view of one of said cartridges shown in Figs. 4a and 4b;
- Fig. 5c: is a side view of one of said cartridges shown in Figs. 4a and 4b;
- Fig. 6a: shows a detailed plan view of a thread fixating means of the present invention in an embodiment with a locking ball;
- Fig. 6b: is a detailed plan view of a fixing means of the present invention in an embodiment with a groove;
- Fig. 6c: is a detailed plan view of a cross section of the shank with a fixing means provided by a groove;
- Figs. 7a-7c: are a cross sectional view, an enlarged cross sectional view and a side view of the shank, respectively, with a fixing means;
- Figs. 8a-8c: are a cross sectional view, an enlarged cross sectional view and a side view of the shank, respectively, with a fixing means;
- Figs. 9a-9g: are side and front views of different embodiments of jaws;
- Figs. 10a-10d: are side views of different embodiments of guiding means;
- Figs. 11a-11e: are side and front views of different embodiments of jaws;
- Figs. 12a-12b: are side and front views of a preferred embodiment of a jaw with guiding means;
- Figs. 13a-13c: are cross sectional views of a jaw corresponding to the embodiment of Fig. 12; and
- Figs. 14a-14f: are side views and cross sectional views of different embodiments, respectively, for mounting guiding means to the shank.

With the apparatus of the present invention, it is possible to deliver the ligature threads into the tissue during preparation. The preparation of the structure where the ligature has to be applied, e.g., a blood vessel, is implemented as follows. The distal end portions of the apparatus, which, in preferred embodiments, form jaws or tips, are applied substantially perpendicularly under the blood vessel wherein, by opening or closing the tips of the apparatus, the structure of the connective tissue is pushed apart and the connective tissue is displaced parallel to the vessel. This procedure is repeated until the vessel is substantially freely accessible. Therefore, the tips of the apparatus can be guides below the substantially freely accessible vessel and the preferably two threads, which have for instance different colours for distinguishing the sides or sizes of the threads, are guided under the vessel. The surgeon or the assistant is able to pick the free ends of the thread which are already placed under the vessel with a tweezers. The threads are then delivered preferably 3 to 5 cm from the tip of the apparatus and the surgeon can pull the apparatus preferably with open tips back while the assistant still bears or fixates the threads so that the threads are placed at the distal and proximal end of the structure. The apparatus can be given away and the surgeon and the assistant is able to knot the ligature at the same time.

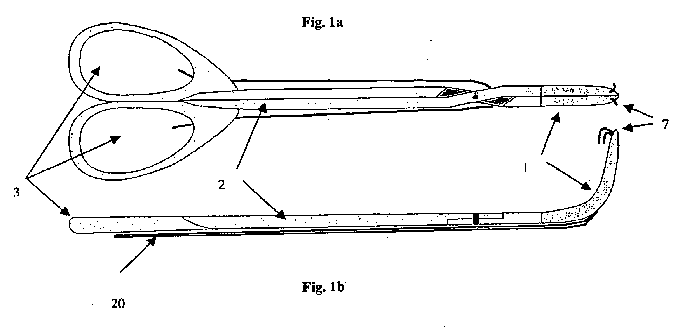

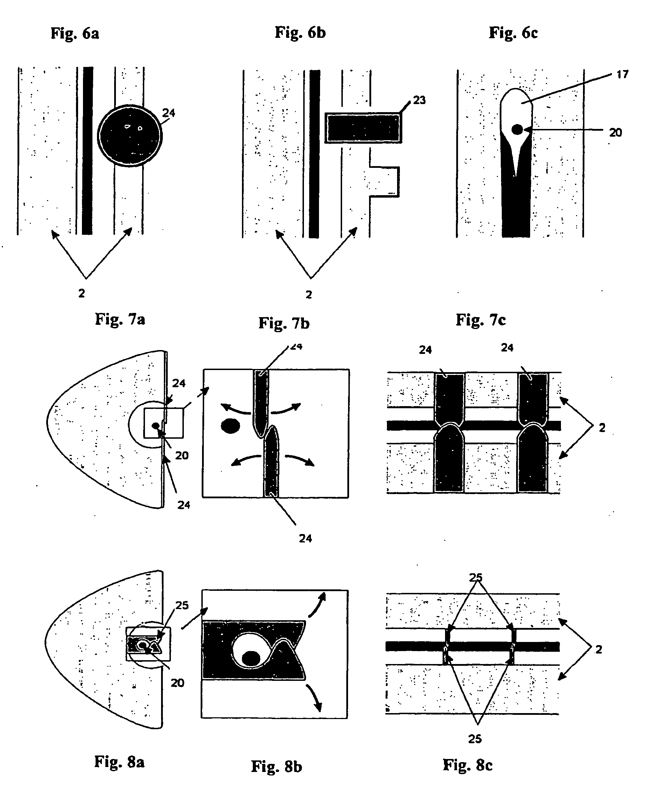

Fig. 1 shows a first preferred embodiment of the present invention wherein the two shanks 2 are connected by a pivot axis 11 to form forceps or Overholt forceps. The proximal ends of this embodiment comprise grip members or ring handles 3 and jaws 1 at the distal end portion wherein the free ends 13 of a thread 20 are delivered by the exit openings 7 at the end or vicinity of the distal end portion 1 of the apparatus. In this embodiment, the threads are guided in a section of the end portion 1 inside the jaws and are guided outside along the shanks. For fixating the threads 20 along the axis of the shanks 2, at least one of the shanks 2 may comprise a recess portion for guiding the thread. Another preferred embodiment for guiding the thread 20 along the shanks 2 and fixing the thread 20 for not sliding away from the shank is shown in Fig. 6. In Fig. 1b, a preferred embodiment of the present invention is shown in side view wherein the distal end portions 1 of the shanks 2 are formed or bent upwardly with respect to the shank 2 looking similar to customary Overholt forceps which is favourable for a surgeon who is generally used to handling this kind of standard instrument.

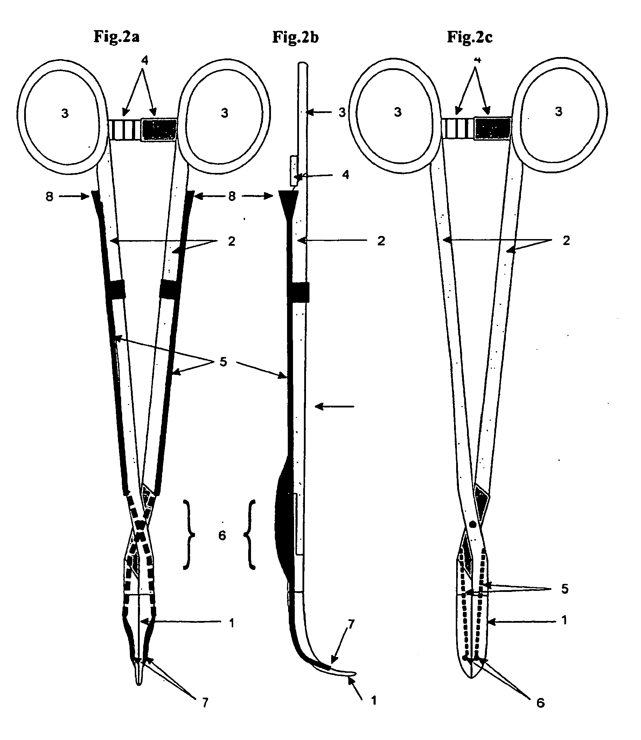

In Figs. 2a and 2b, the schematic front and side views of another preferred embodiment of the present invention is shown wherein in comparison to Fig. 1 an additional guiding means 5 or thread duct along the shanks 2 is applied. In this embodiment, the guiding means 5 is either fixed to or removable from the shanks 2. The guiding means 5 may be made of metal or plastic or any other material which is easily mouldable and is preferably a closed guiding means to prevent the thread (not shown) from contamination. In this embodiment, the guiding means 5 comprises a thread inlet opening 8 wherein the thread is inserted into the guiding means. The guiding means 5 is heightened at the thread crossing section 6 in the vicinity of the pivot axis 12 for guiding the thread beneath the shanks 2. Furthermore, the thread may be guided by the additional guiding means which are attached to the shanks along the distal end portion, preferably formed like a pipe, tube or conduit, and preferably comprises exit openings 7 for dispensing the free end 13 of the thread. Again, in side view of Fig. 2b, the apparatus comprises a distal end portion 1 which is formed or bent upwardly with respect to the shanks 2 to allow the threads to cross each other and to allow the two shanks to be pivoted. This apparatus, looking quite similar to an Overholt forceps, may optionally comprise a ratchet 4 for fixating the distance of the jaws or spring parts to provide an additional supporting force for converging or diverging the shanks. Fig. 2c shows a similar embodiment of the present invention, without the guiding means along the shanks 2 but with a guiding means 5 at the distal end portions.

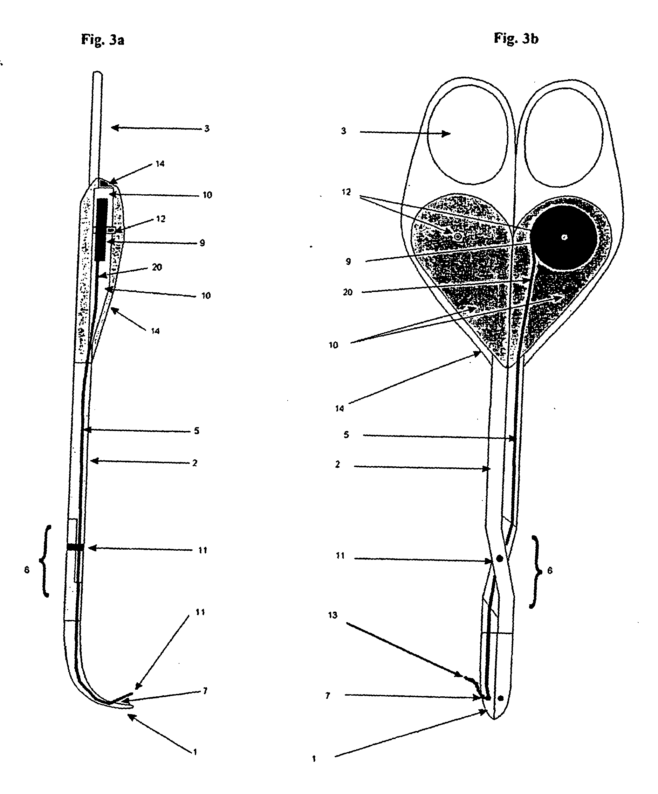

Figs. 3a and 3b show another preferred embodiment of the present invention, wherein, in comparison with the previous Figure, the apparatus comprises an additional thread storage means 10 for providing enough thread for a plurality of ligatures. The thread storage means in this preferred embodiment of the invention is attached next to the grip members 3 and comprises a cover 14 to prevent the storage means from contamination. Again, the thread is guided by a guiding means 5 along the shanks 2, wherein in this embodiment the guiding means is inside the shank 2. The thread storage means applied to at least one shank may be either a permanently mounted means of the grip members or may be removable or disposable in part. Furthermore, each thread storage means at the corresponding shank may comprise one or more threads wherein the threads may either be stored in folded manner inside the storage means or wound up around a thread spool 9 which is rotatably assembled inside the thread storage means on a spool axis 12. Depending on the material of the apparatus and the thread storage means, this apparatus could be used as a disposal apparatus or parts of the storage means are removable to disinfect the whole apparatus or parts of the apparatus.

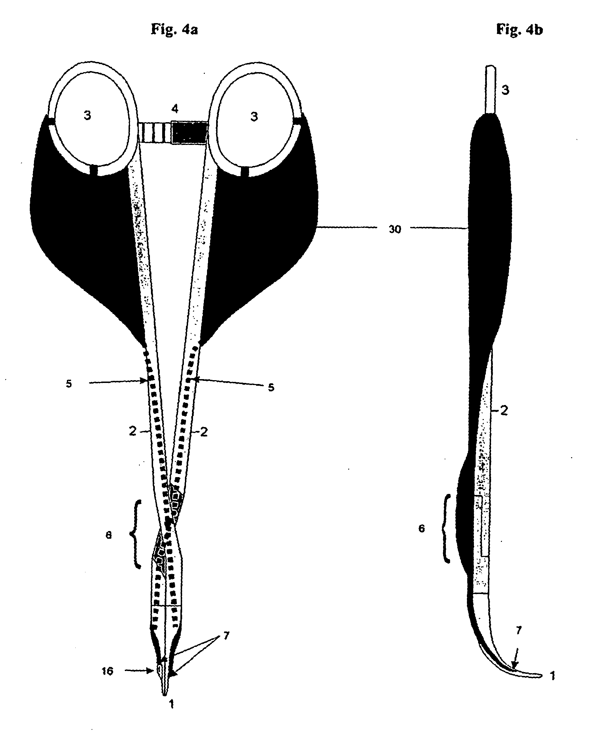

Figs. 4a and 4b show, the schematic front and side views of another preferred embodiment of the present invention, wherein the thread storage means together with the guiding means is implemented in a removable cartridge 30. The apparatus without the cartridge 30 is quite similar to a conventional or Overholt forceps but with additional holes or recess portions or other notches for fixating the cartridges 30. In this embodiment, the cartridge 30 additionally comprises a thread guiding means 5 in form of a pipe, tube or conduit which is attachable to or into the shanks 2. The cartridge and the thread ducts or guiding means can be easily attached to and removed from the shanks as needed to perform a ligature and to sterilize said duct or to replace damaged ones. Furthermore, it is apparent that the guiding means or other components may also be easily disconnected and removed from the shanks for replacement, sterilization or disposal. As can bee seen in Fig. 4b, the guiding means is heightened or provides an additional space or curvature at the thread crossing portion and comprises the exit opening 7 for dispensing the thread at the jaw 1 in the vicinity of the tips.

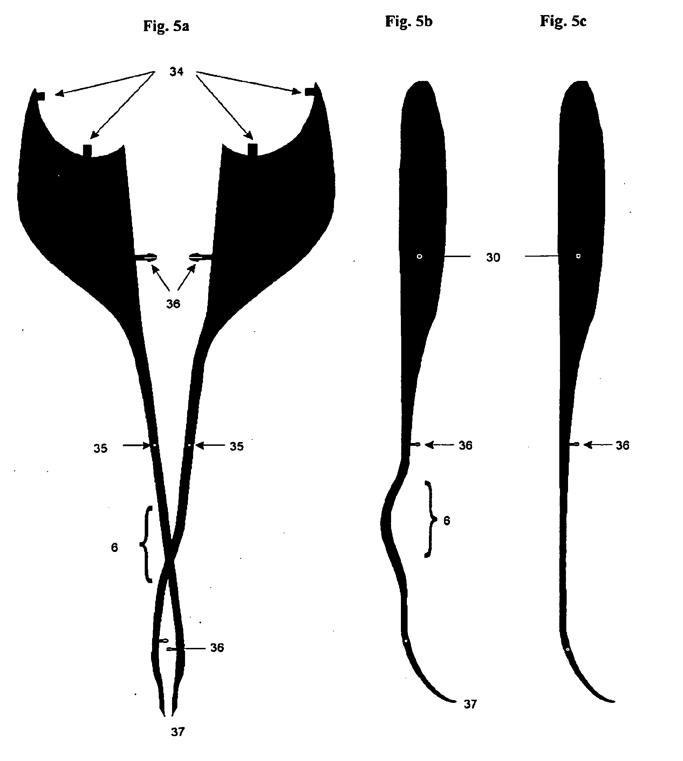

In Fig. 5a, the cartridge 30 together with the thread duct 35 is shown in the front view and Figs. 5b and 5c show the cartridge in the side view with different shapes of the thread duct 35 for guiding the thread around the pivot axis or lap joint and to allow the shanks to be pivoted. The cartridges 30 are preferably attached or snap fitted to the apparatus by clips 34 and/or push buttons 36. Another preferred attaching or mounting mechanism comprises a plurality of holes or slits in the shank or grip members in which the corresponding projections 36 of substantially cylindrical shape of the duct 24 are snap fitted. It is to be understood that also different push buttons or clips are possible. The advantage of this cartridge is the easy and separate disinfection of the apparatus part without the cartridge and the easy and cheap manufacture of the cartridges which may comprise thread enough for a plurality of ligatures in a disposable manner. The cartridge together with the thread guiding means is preferably only used for one incision and the small diameter pipes or ducts or the guiding means need not be disinfected after use.

Figs. 6a to 6c show different preferred embodiments of the present invention for fixating/bearing the thread in the guiding means inside the shank 2 preventing a loosing of the thread during handling. In Fig. 6a, the thread is retained in the guiding means by the locking ball 24, wherein the locking ball 24 is fixed, rotatably or moveably attached to the shank 2 and bears a force against the thread 20 so that the thread 20 is only movable inside the guiding means when a adequate force is applied to the free end of the thread. For instance the surgeon may apply this adequate force by pulling at the free ends with tweezers or another forceps. Fig. 6b shows another preferred embodiment with a thread maintaining, fixating or bearing means, wherein the thread 20 is again guided in the guiding means inside the shank 2 and the inner wall of the shank 2 comprises an open groove 22 in which a tongue 23 pushes the thread against the inner wall of the guiding means for fixating the thread. The tongue 23 could be either an independent tongue which is arranged movably with respect to the groove 22, wherein a spring pushes the tongue 23 against the thread 20 for bedding or bearing the thread. In another preferred embodiment of the invention, the tongue 23 may be attached at the corresponding place of the other shank of the apparatus wherein the tongue 23 is imposed into the groove 22 by closing the forceps and presses the tongue 23 against the thread 20 for fixating.

Another preferred embodiment for maintaining or bearing the thread 20 inside the shank 2 is shown in Fig. 6c, wherein the thread 20 is guided inside the shank 2, and a groove part 17 with a tapered groove is able to fixate the thread 20 by clamping the thread inside the tapered groove.

In Fig. 7a, another preferred embodiment is shown in a cross sectional side view wherein the guiding duct is only partly inside the shank and/or the end portion of the shank. Therefore, the guiding duct for the thread is partly open. To prevent the thread 20 from falling out of this guiding duct, flaps 24 are pair wise attached to the shank and are overlapping partly in the region of the guiding duct. In Fig. 7b, an enlarged view of the thread 20 with the flaps 24 is shown, wherein the arrows indicate the movement of the flaps when the thread is implemented or removed from the guiding duct. The flaps are preferably made of metal or any other flexible material, wherein the elasticity of the flaps pushes the pair wise flaps against each other and prevents therefore the thread from loosing out of the duct. In Fig. 7c, the same construction is shown in a front view with two pairs of flaps 4 attached to the shank 24, wherein the overlap of the pair wise flaps can be seen.

Another preferred embodiment of a guiding means with a partly open guiding duct is shown in Figs. 8a to 8c. Again, Fig. 8a is a cross sectional view of the shank 2 with a partly open guiding duct, wherein in this embodiment the thread 20 is prevented from loosing by at least one clamp 25. This clamp 25 may be made of one piece or produced pair wise as shown before. In contrast to the previous embodiment, the overlapping of the clamp is perpendicular to the direction of the guiding duct. For applying the thread 20 into this guiding duct formed by the clamp 25, the two ends of the clamp have to be shifted upwardly or correspondingly downwardly until the duct inside the clamp 25 is freely accessible. This movement of the ends of the clamp 25 is shown in Fig. 8b, which is an enlargement view of Fig. 8a. The two arrows are indicating the movement of the clamp ends when the thread is implemented or removed from the guiding duct. After applying the thread into the duct inside the clamps ends of the clamps will spring back and will guide the thread 20 inside the notch of the clamp. In Fig. 8c, the same construction is shown in a side view wherein it can be clearly seen that the overlapping of the two ends of the clamps 25 are perpendicular to the thread guiding direction in contrast to Fig. 7c, wherein the overlapping is in the direction of the guiding of the thread 20.

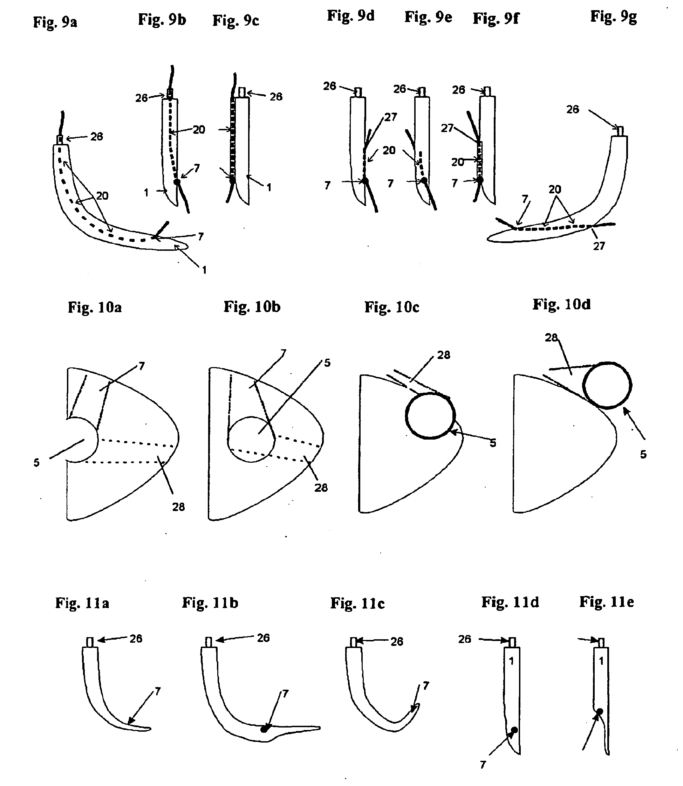

In the following Figs. 9a to 9g, different embodiments of the shank end portion or jaw are shown with different embodiments for guiding the thread either inside the jaw, partly inside the jaw or outside the jaw by an additional guiding means. Furthermore, different embodiments for placing the exit opening 7 at the vicinity of the end portion or jaw are shown. Fig. 9a shows an end portion in the embodiment of a bent jaw 1, wherein this jaw is removable from the shank 2 and can be mounted to the shank by the threaded protrusion 26. The dashed line indicates the path of the thread inside the jaw. The guiding into the jaw occurs through the threaded protrusion 26 which is hollow inside for guiding the suture thread. The exit opening 7 is placed in the vicinity of the tip of the jaw 1. It is obvious to those skilled in the art that different shapes of the jaw are possible.

Fig. 9b shows the same embodiment as Fig. 9a in the front view. The thread 20 is routed by different fixating means as shown, e.g., in Figs. 7 to. 8. The exit opening 7 is applied at the straight edge of the jaw which is preferably the inner side of the jaw opposite to the jaw of the other shank. Fig. 9c again shows a front view of a jaw but in this embodiment the thread 20 is guided not central inside the jaw but outside the jaw and, in this embodiment, the exit opening 7 is placed at the curved edge of the jaw which defines the outside direction or outer part of the apparatus. Figs. 9d to. 9f show the front view of different embodiments, wherein the thread 20 is guided only partly by the jaw either central in the inner part of the jaw, i.e., the straight edge, central inside the jaw Fig. 9e or at the outside part, i.e., curved edge Fig. 9f. Fig. 9g shows a side view of a jaw 1 accordingly to the front view of Figs. 9d to 9f, wherein the thread 20 is brought into the guiding means of the jaw by the input opening 27 which is, in this preferred embodiment, in the middle part of the jaw 1. In Figs. 9c to 9g, the thread is not guided through the threaded protrusion 26 contrary to Figs. 9a to 9b and, therefore, the threaded protrusion of the embodiments 9c to 9g may be solid and easier to produce. In Fig. 9c, wherein the guiding of the thread 20 is at the outer part by an additional guiding duct, the thread 20 may be guided to the shank 2 outside of threaded protrusion 26.

Figs. 10a to 10d show different embodiments with different positions of the guiding means 5 at the jaw of the apparatus wherein Fig. 9a shows the guiding means partly inside the jaw, Fig. 10b inside the jaw, Fig. 10c an additional duct attached partly inside the jaw and Fig. 10d a duct attached outside the jaw 1. The cross sectional side view of all four Figures show the guiding means 5, the exit opening 7 and the input opening 27 which is partly indicated by dashed lines.

Figs. 11a to 11e show side views of different jaw shapes. Fig. 11a shows the side view with a classical shape of a jaw, wherein the tip is bent around 90° and the jaw is tapered into the direction of the tip. Again, all the embodiments of the jaw shown in Figs. 11a to 11e are removable end portions which can be mounted or applied to the shanks by the winding 26. Fig. 11b shows a modified shape from Fig. 11a, wherein the jaw is bent once more about 90° but is only tapered at the very end section next to the tip. Fig. 11e shows an embodiment of a jaw which is preferably for applying ligatures and vessels at the vicinity of a surface (skin) light removing varices. The jaw is bent preferably approximately 160° and is tapered into the direction of the tip. This shape of jaw allows circumscribing nearly the whole vessel by the jaw. Figs. 11d and 11e show the front view of two different embodiments of the jaw, wherein Figure 11d shows a classical shape of a jaw like in common Overholt forceps. Fig. 11e shows a jaw which is only tapered strongly in the vicinity of the tip. Of course, the exit opening 7 can be applied at different positions to all embodiments shown.

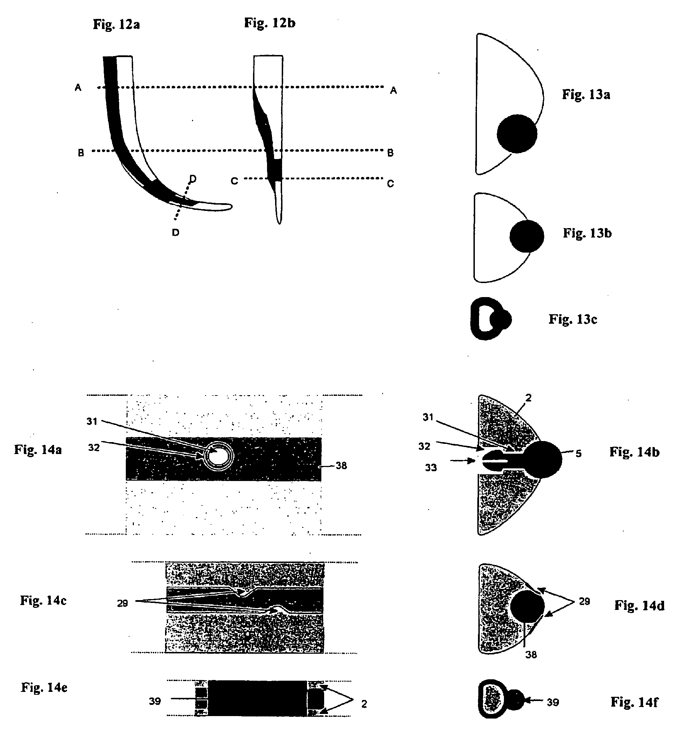

Figs 12a and 12b show the jaw 1 in a side and front view with a guiding duct, indicated by the dark region, which is attached partly at the outer side of the jaw. The dashed lines AA, BB, CC, DD indicate planes for cross sectional views shown in Figs. 13a to 13c.

Figs. 14a to 14f show different embodiments for mounting the guiding means 5 to the shank 2. Fig. 14a shows the side view of the shank 2, wherein the dark grey area corresponds to the counter sink groove. For applying the guiding means by push buttons, one hole is comprising two different diameters. The hole 31,which is the outer hole, with a smaller diameter and the inner hole 32 with a larger diameter than the outer hole 31. In the corresponding side view in Fig. 14b, the guiding means 5 is snap fitted to the shank 2, wherein the push button 32 is pushed through the smaller outer hole 31 and the larger part of the push button 33 gets stuck in the larger inner hole 32. Fig. 14c shows another preferred embodiment for mounting the guiding duct to the shank 2, wherein Fig. 14d is a corresponding cross sectional view. In this embodiment, the guiding duct 5 is mounted and fixated by the clips 29. The groove is counter sink deep enough to include half or more of the diameter of the guiding duct. In this embodiment, the guiding duct is preferably made of elastic material like plastics as during pushing the guiding means into the groove 38 the guiding means is squeezed for short time during mounting. Fig. 14e shows again a side view of another preferred embodiment for applying the guiding means to the shank. Fig. 14f shows the corresponding cross sectional view of this embodiment. Again, the dark grey areas show the counter sink groove for carrying the guiding means. For fixating the guiding means, a nose 39 is applied outside of the guiding means and bears the guiding means next to the shank 2. It should be noted that either only one of the previous shown embodiments may be applied or a combination of more than one embodiment. For example, the mounting according to 14a and 14b is a preferred embodiment along the shanks in the middle part of the apparatus and the mounting according to Figs. 14e and 14f may be preferred for mounting the guiding duct at the end portions of the shanks and in the vicinity of the tips.

The detailed description above is intended only to illustrate certain preferred embodiments of the present invention. It is in no way intended to limit the scope of the invention as set out in the claims.

## Claims

1. An apparatus for the placement and controlled manipulation of at least two ligatures, comprising
at least two shanks (2) each having a proximal end and a distal end, wherein the shanks are hinged to each other such they define forceps; and
at least one ligature guiding means associated with each shank, each guiding means having an exit (7) at or in the vicinity of the respective shank distal end;
wherein further the distal ends are each curved in a plane generally perpendicular to the plane defined by the proximal portions of the hinged shanks, and each exit (7) is adapted for dispensing the free end of a ligature generally within the plane of the curved distal end of the respective shank.

2. The apparatus according to claim 1, wherein the upwardly formed ends are removable from the shanks (2).

3. The apparatus according to claim 1 or 2, wherein the apparatus comprises at least one ligature thread (20).

4. The apparatus according to claim 3, wherein the shanks are movably connected and a spatial distance between the distal end portions (1) can be varied.

5. The apparatus according to claim 1, 2 3, or 4, wherein the shanks (2) are connected by a pivot axis (11) to form a forceps.

6. The apparatus according to claim 1, 2, 3 4, or 5, wherein the shanks (2) are connected by a spring part.

7. The apparatus according to any one of the preceding claims, wherein the distal end portion of at least one shank forms a jaw (1).

8. The apparatus according to any one of the preceding claims, wherein at least one shank (2) has a recess portion for guiding the thread (20) along the axis of said shank (2).

9. The apparatus according to any one of the preceding claims, wherein at least one fixating means prevents the at least one thread (20) to slide away from the guiding means.

10. The apparatus according to any one of the preceding claims, wherein at least one shank (2) comprises a grip member (3) at the proximal end portion.

11. The apparatus according to any one of the preceding claims, wherein at least one thread storage means (19) is attached to the proximal end portion.

12. The apparatus according to claim 11, wherein at least one thread spool (9) is applied to said thread storage means (19).

13. The apparatus according to claim 11, comprising at least one thread which is folded in said storage means (19).

14. The apparatus according to any one of the preceding claims, wherein the guiding means guides at least one thread (20) in a duct
a) inside the shank (2),
b) partly inside the shank (2),
c) outside the shank (2), and/or
d) in an additional duct which is attached to the shank (2).

15. The apparatus according any one of the preceding claims, wherein two shanks are connected by a pivot axis (11) and the at least two threads are not crossing in the environment of the pivot axis (11), at least one thread (20) is guided either at least
a) inside one(said) shank (2),
b) partly inside one(said) shank (2),
c) outside one(said) shank (2), and/or
d) in at least one additional duct which is attached to said shank (2).

16. The apparatus according any one of claims 1 to 13, wherein two shanks are connected by a pivot axis (11) and the at least two threads are crossing at a crossing section (6) in the environment of the pivot axis (11), at least one thread (20) being guided either at least
a) inside one(said) shank (2),
b) partly inside one(said) shank (2),
c) outside one(said) shank (2), and/or
d) in at least one additional duct which is attached to said shank (2).

17. The apparatus according to claim 16, wherein the additional duct is removable.

18. The apparatus according to any one of the preceding claims, wherein the guiding means at the distal end portion guides the at least one thread (20) either
a) inside,
b) partly inside,
c) outside, and/or
d) in an additional duct which is attached to the distal end portion.

19. The apparatus according to claim 18 d), wherein the additional duct is removable.

20. The apparatus according to any one of the preceding claims, wherein the exit (7) at the distal end portion or in the vicinity of the distal end portion dispenses the free-end (13) of the thread (20) either from the
a) inner,
b) middle, and/or
c) outer
side of the end portion.

21. The apparatus according to any one of the preceding claims, wherein the end portion (1) of the distal end is either a
a) straight, and/or
b) curved portion.

22. The apparatus according to any one of the preceding claims, wherein the end portion (1) of the distal end is either
a) a rigid, and/or
b) a flexible
material.

23. The apparatus according to any one of the preceding claims, wherein the thread (20) is prevented from slipping out of the guiding means either by
a) grooves (17),
b) at least one groove (22) at one of the shanks (2) and a tongue (23) at least at another shank (2) at the corresponding part to clamp the thread (20) inside the groove (22) by the corresponding tongue (23) when the shanks are close to each other,
c) clips made of metal or plastic, and/or
d) fixing means like a spring or a locking ball (24).

24. The apparatus according to any one of the preceding claims, further comprising a cartridge (30) with:
a thread storage means (21) for providing a thread (20);
wherein the cartridge (30) is applicable for providing said thread (20) for guiding by the guiding means of the apparatus, wherein at least one thread spool (9) is rotatably mountable within the thread storage means (21).

25. The apparatus according to claim 24, wherein the cartridge (30) is removable.

26. The apparatus according to one of claims 24 and 25, wherein the thread spool (9) comprises at least one thread (20).

27. The apparatus according to any one of claims 24-26, wherein the cartridge (30) comprises at least one push button (36) for mounting the cartridge (30) on the apparatus.

28. The apparatus according to any one of claims 24-27, wherein the cartridge comprises a thread guiding means for guiding the thread (20) along a shank (2).

29. The apparatus according to any one of claims 24-28
wherein the at least one thread (20) is provided by the thread storage means (21) of the cartridge (30);
and the at least one thread (20) is guided by the guiding means and dispensed through an exit (7).

## Patentansprüche

1. Vorrichtung zur Platzierung und gesteuerten Manipulation von mindestens zwei Ligaturen, mit:
zumindest zwei Schenkeln (2) mit jeweils einem proximalen Ende und einem distalen Ende, wobei die Schenkel derartig gelenkig miteinander verbunden sind, daß sie eine Zange bilden; und
zumindest einer Ligaturführungseinrichtung, die jedem Schenkel zugeordnet ist, wobei jede Führungseinrichtung einen Ausgang (7) an dem oder in der Nähe des jeweiligen distalen Schenkelendes hat;
wobei ferner die distalen Enden jeweils in einer Ebene im allgemeinen senkrecht zu der Ebene, die durch die proximalen Abschnitte der gelenkig verbundenen Schenkel definiert sind, gekrümmt sind und jeder Ausgang (7) zum Abgeben des freien Endes einer Ligatur im allgemeinen in der Ebene des gekrümmten distalen Endes des jeweiligen Schenkels eingerichtet ist.

2. Vorrichtung nach Anspruch 1, wobei die nach oben geformten Enden von den Schenkeln (2) entfernbar sind.

3. Vorrichtung nach Anspruch 1 oder 2, wobei die Vorrichtung zumindest einen Ligaturfaden (20) aufweist.

4. Vorrichtung nach Anspruch 3, wobei die Schenkel beweglich verbunden sind und ein räumlicher Abstand zwischen den distalen Endabschnitten (1) variiert werden kann.

5. Vorrichtung nach Anspruch 1, 2, 3 oder 4, wobei die Schenkel (2) durch eine Drehachse (11) verbunden sind, um eine Zange zu bilden.

6. Vorrichtung nach Anspruch 1, 2, 3, 4 oder 5, wobei die Schenkel (2) durch ein Federteil verbunden sind.

7. Vorrichtung nach einem der vorhergehenden Ansprüche, wobei der distale Endabschnitt zumindest eines Schenkels eine Backe (1) bildet.

8. Vorrichtung nach einem der vorhergehenden Ansprüche, wobei zumindest ein Schenkel (2) einen Vertiefungsabschnitt zum Führen des Fadens (20) entlang der Achse des Schenkels (2) hat.

9. Vorrichtung nach einem der vorhergehenden Ansprüche, wobei zumindest eine Fixierungseinrichtung verhindert, daß der zumindest eine Faden (20) der Führungseinrichtung entgleitet.

10. Vorrichtung nach einem der vorhergehenden Ansprüche, wobei zumindest ein Schenkel (2) ein Griffteil (3) am proximalen Endabschnitt aufweist.

11. Vorrichtung nach einem der vorhergehenden Ansprüche, wobei zumindest eine Fadenaufbewahrungseinrichtung (19) am proximalen Endabschnitt angebracht ist.

12. Vorrichtung nach Anspruch 11, wobei zumindest eine Fadenspule (9) bei der Fadenaufbewahrungseinrichtung (19) Anwendung findet.

13. Vorrichtung nach Anspruch 11, mit zumindest einem Faden, der in der Aufbewahrungseinrichtung (19) gefaltet ist.

14. Vorrichtung nach einem der vorhergehenden Ansprüche, wobei die Führungseinrichtung zumindest einen Faden (20) in einem Kanal
a) innerhalb des Schenkels (2),
b) teilweise innerhalb des Schenkels (2),
c) außerhalb des Schenkels (2) und/oder
d) in einem zusätzlichen Kanal, der am Schenkel (2) angebracht ist,
führt.

15. Vorrichtung nach einem der vorhergehenden Ansprüche, wobei zwei Schenkel durch eine Drehachse (11) verbunden sind und die zumindest zwei Fäden sich nicht in der Nähe der Drehachse (11) kreuzen, wobei zumindest ein Faden (20) zumindest
a) innerhalb eines (des) Schenkels (2),
b) teilweise innerhalb eines (des) Schenkels (2),
c) außerhalb eines (des) Schenkels (2) und/oder
d) in zumindest einem zusätzlichen Kanal, der an dem Schenkel (2) angebracht ist,
geführt wird.

16. Vorrichtung nach einem der Ansprüche 1 bis 13, wobei zwei Schenkel durch eine Drehachse (11) verbunden sind und die zumindest zwei Fäden sich an einem Kreuzungsteilstück (6) in der Umgebung der Drehachse (11) kreuzen, wobei zumindest ein Faden (20) zumindest
a) innerhalb eines (des) Schenkels (2),
b) teilweise innerhalb eines (des) Schenkels (2),
c) außerhalb eines (des) Schenkels (2) und/oder
d) in zumindest einem zusätzlichen Kanal, der an dem Schenkel (2) angebracht ist,
geführt wird.

17. Vorrichtung nach Anspruch 16, wobei der zusätzliche Kanal entfernbar ist.

18. Vorrichtung nach einem der vorhergehenden Ansprüche, wobei die Führungseinrichtung am distalen Endabschnitt den zumindest einen Faden (20)
a) innen,
b) teilweise innen,
c) außen und/oder
d) in einem zusätzlichen Kanal, der am distalen Endabschnitt angebracht ist,
führt.

19. Vorrichtung nach Anspruch 18d), wobei der zusätzliche Kanal entfernbar ist.

20. Vorrichtung nach einem der vorhergehenden Ansprüche, wobei der Ausgang (7) am distalen Endabschnitt oder in der Nähe des distalen Endabschnitts das freie Ende (13) des Fadens (20) von der
a) Innen-,
b) Mittel- und/oder
c) Außenseite des Endabschnitts abgibt.

21. Vorrichtung nach einem der vorhergehenden Ansprüche, wobei der Endabschnitt (1) des distalen Endes ein
a) gerader und/oder
b) gekrümmter Abschnitt ist.

22. Vorrichtung nach einem der vorhergehenden Ansprüche, wobei der Endabschnitt (1) des distalen Endes
a) ein starres und/oder
b) ein flexibles
Material ist.

23. Vorrichtung nach einem der vorhergehenden Ansprüche, wobei das Herausrutschen des Fadens (20) aus der Führungseinrichtung verhindert wird durch
a) Nuten (17),
b) zumindest eine Nut (22) an einem der Schenkel (2) und eine Zunge (23) zumindest an einem anderen Schenkel (2) an dem entsprechenden Teil, um den Faden (20) innerhalb der Nut (22) durch die entsprechende Zunge (23) festzuklemmen, wenn die Schenkel nahe aneinander sind,
c) Klammern, die aus Metall oder Kunststoff bestehen, und/oder
d) eine Fixierungseinrichtung wie eine Feder oder eine Verriegelungskugel (24).

24. Vorrichtung nach einem der vorhergehenden Ansprüche, ferner mit einer Patrone (30) mit:
einer Fadenaufbewahrungseinrichtung (21) zur Bereitstellung eines Fadens (20);
wobei die Patrone (30) zur Bereitstellung des Fadens (20) zum Führen durch die Führungseinrichtung der Vorrichtung anwendbar ist, wobei zumindest eine Fadenspule (9) in der Fadenaufbewahrungseinrichtung (21) drehbar angeordnet werden kann.

25. Vorrichtung nach Anspruch 24, wobei die Patrone (30) entfernbar ist.

26. Vorrichtung nach einem der Ansprüche 24 und 25, wobei die Fadenspule (9) zumindest einen Faden (20) aufweist.

27. Vorrichtung nach einem der Ansprüche 24 bis 26, wobei die Patrone (30) zumindest eine Drucktaste (36) zum Anordnen der Patrone (30) an der Vorrichtung aufweist.

28. Vorrichtung nach einem der Ansprüche 24 bis 27, wobei die Patrone eine Fadenführungseinrichtung zum Führen des Fadens (20) entlang eines Schenkels (2) aufweist.

29. Vorrichtung nach einem der Ansprüche 24 bis 28,
wobei der zumindest eine Faden (20) durch die Fadenaufbewahrungseinrichtung (21) der Patrone (30) bereitgestellt wird;
und der zumindest eine Faden (20) durch die Führungseinrichtung geführt und durch den Ausgang (7) abgegeben wird.

## Revendications

1. Appareil pour la mise en place et la manipulation contrôlée d'au moins deux ligatures, comprenant :
au moins deux tiges (2) ayant chacune une extrémité proximale et une extrémité distale, dans lequel les tiges sont articulées entre elles de sorte qu'elles définissent une pince ; et
au moins un moyen de guidage de ligature associé avec chaque tige, chaque moyen de guidage ayant une sortie (7) au niveau de ou à proximité de l'extrémité distale de la tige respective ;
dans lequel, en outre, les extrémités distales sont chacune incurvées dans un plan généralement perpendiculaire au plan défini par les parties proximales des tiges articulées, et chaque sortie (7) est adaptée pour distribuer l'extrémité libre d'une ligature généralement dans le plan de l'extrémité distale incurvée de la tige respective.

2. Appareil selon la revendication 1, dans lequel les extrémités formées de manière ascendante sont amovibles des tiges (2).

3. Appareil selon la revendication 1 ou 2, dans lequel l'appareil comprend au moins un fil de ligature (20).

4. Appareil selon la revendication 3, dans lequel les tiges sont raccordées de manière mobile et la distance spatiale entre les parties d'extrémité distale (1) peut être modifiée.

5. Appareil selon la revendication 1, 2, 3 ou 4, dans lequel les tiges (2) sont raccordées par un axe de pivot (11) pour former une pince.

6. Appareil selon la revendication 1, 2, 3, 4 ou 5, dans lequel les tiges (2) sont raccordées par une partie de ressort.

7. Appareil selon l'une quelconque des revendications précédentes, dans lequel la partie d'extrémité distale d'au moins une tige forme une mâchoire (1).

8. Appareil selon l'une quelconque des revendications précédentes, dans lequel au moins une tige (2) a une partie d'évidement pour guider le fil (20) le long de l'axe de ladite tige (2).

9. Appareil selon l'une quelconque des revendications précédentes, dans lequel au moins un moyen de fixation empêche le au moins un fil (20) de sortir en glissant des moyens de guidage.

10. Appareil selon l'une quelconque des revendications précédentes, dans lequel au moins une tige (2) comprend un élément de préhension (3) au niveau de la partie d'extrémité proximale.

11. Appareil selon l'une quelconque des revendications précédentes, dans lequel au moins un moyen de stockage de fil (19) est fixé à la partie d'extrémité proximale.

12. Appareil selon la revendication 11, dans lequel au moins une bobine de fil (9) est appliquée audit moyen de stockage de fil (19).

13. Appareil selon la revendication 11, comprenant au moins un fil qui est plié dans ledit moyen de stockage (19).

14. Appareil selon l'une quelconque des revendications précédentes, dans lequel le moyen de guidage guide au moins un fil (20) dans un conduit,
a) à l'intérieur de la tige (2),
b) partiellement à l'intérieur de la tige (2),
c) à l'extérieur de la tige (2), et/ou
d) dans un conduit supplémentaire qui est fixé à la tige (2).

15. Appareil selon l'une quelconque des revendications précédentes, dans lequel deux tiges sont raccordées par un axe de pivot (11) et les au moins deux fils ne se croisent pas dans l'environnement de l'axe de pivot (11), au moins un fil (20) est guidé au moins :
a) à l'intérieur d'une (de ladite) tige (2),
b) partiellement à l'intérieur d'une (de ladite) tige (2),
c) à l'extérieur d'une (de ladite) tige (2), et/ou
d) dans au moins un conduit supplémentaire qui est fixé à ladite tige (2).

16. Appareil selon l'une quelconque des revendications 1 à 13, dans lequel deux tiges sont raccordées par un axe de pivot (11) et les au moins deux fils se croisent au niveau d'une section de croisement (6) dans l'environnement de l'axe de pivot (11), au moins un fil (20) étant guidé au moins :
a) à l'intérieur d'une (de ladite) tige (2),
b) partiellement à l'intérieur d'une (de ladite) tige (2),
c) à l'extérieur d'une (de ladite) tige (2), et/ou
d) dans au moins un conduit supplémentaire qui est fixé à ladite tige (2).

17. Appareil selon la revendication 16, dans lequel le conduit supplémentaire est amovible.

18. Appareil selon l'une quelconque des revendications précédentes, dans lequel le moyen de guidage au niveau de la partie d'extrémité distale, guide le au moins un fil (20), soit :
a) à l'intérieur,
b) partiellement à l'intérieur,
c) à l'extérieur, et/ou
d) dans un conduit supplémentaire qui est fixé à la partie d'extrémité distale.

19. Appareil selon la revendication 18 d), dans lequel le conduit supplémentaire est amovible.

20. Appareil selon l'une quelconque des revendications précédentes, dans lequel la sortie (7) au niveau de la partie de l'extrémité distale ou à proximité de la partie d'extrémité distale distribue l'extrémité libre (13) du fil (20) à partir :
a) du côté interne,
b) du côté central, et/ou
c) du côté extérieur de la partie d'extrémité.

21. Appareil selon l'une quelconque des revendications précédentes, dans lequel la partie d'extrémité (1) de l'extrémité distale est :
a) une partie droite, et/ou
b) une partie incurvée.

22. Appareil selon l'une quelconque des revendications précédentes, dans lequel la partie d'extrémité (1) de la partie distale est :
a) un matériau rigide, et/ou
b) un matériau flexible.

23. Appareil selon l'une quelconque des revendications précédentes, dans lequel on empêche le fil (20) de sortir en glissant du moyen de guidage, soit par :
a) des rainures (17),
b) au moins une rainure (22) au niveau de l'une des tiges (2) et une languette (23) au moins au niveau d'une autre tige (2) au niveau de la partie correspondante pour serrer le fil (20) à l'intérieur de la rainure (22) par la languette (23) correspondante lorsque les tiges sont proches l'une de l'autre,
c) des pinces en métal ou en plastique, et/ou
d) des moyens de fixation comme un ressort ou une bille de verrouillage (24).

24. Appareil selon l'une quelconque des revendications précédentes, comprenant en outre une cartouche (30) avec :
un moyen de stockage de fil (21) pour fournir un fil (20) ;
dans lequel la cartouche (30) est applicable pour fournir ledit fil (20) pour le guidage par les moyens de guidage de l'appareil, et dans lequel au moins une bobine de fil (9) peut être montée de manière rotative dans le moyen de stockage de fil (21).

25. Appareil selon la revendication 24, dans lequel la cartouche (30) est amovible.

26. Appareil selon l'une quelconque des revendications 24 et 25, dans lequel la bobine de fil (9) comprend au moins un fil (20).

27. Appareil selon l'une quelconque des revendications 24 à 26, dans lequel la cartouche (30) comprend au moins un bouton poussoir (36) pour monter la cartouche (30) sur l'appareil.

28. Appareil selon l'une quelconque des revendications 24 à 27, dans lequel la cartouche comprend un moyen de guidage de fil pour guider le fil (20) le long d'une tige (2).

29. Appareil selon l'une quelconque des revendications 24 à 28, dans lequel le au moins un fil (20) est fourni par le moyen de stockage de fil (21) de la cartouche (30) ;
et le au moins un fil (20) est guidé par le moyen de guidage et distribué par une sortie (7).
